(19) Europäisches Patentamt
European Patent Office
Office européen des brevets

(11) Veröffentlichungsnummer: **0 510 562 A1**

# (12) EUROPÄISCHE PATENTANMELDUNG

(21) Anmeldenummer: **92106743.5**

(51) Int. Cl.5: **C07D 237/20**, A61K 31/50

(22) Anmeldetag: **21.04.92**

(30) Priorität: **26.04.91 CH 256/91**
**26.04.91 CH 255/91**
**05.07.91 CH 997/91**

(43) Veröffentlichungstag der Anmeldung:
**28.10.92 Patentblatt 92/44**

(84) Benannte Vertragsstaaten:
**PT**

(71) Anmelder: **Byk Gulden Lomberg Chemische Fabrik GmbH**
**Byk-Gulden-Strasse 2**
**W-7750 Konstanz(DE)**

(72) Erfinder: **Amschler, Hermann, Dr.**
**Hohenhewenstrasse 19**
**W-7760 Radolfzell(DE)**
Erfinder: **Figala, Volker, Dr.**
**Am Hochfirst 2**
**W-7753 Allensbach 4(DE)**
Erfinder: **Kilian, Ulrich, Dr.**
**Ackersweg 26**
**W-7753 Allensbach(DE)**
Erfinder: **Beume, Rolf, Dr.**
**Bohlstrasse 13**
**W-7750 Konstanz(DE)**
Erfinder: **Riedel, Richard, Dr.**
**Durlesbach 7**
**W-7967 Bad Waldsee(DE)**
Erfinder: **Eltze, Manfrid, Dr.**
**Schützenstrasse 20**
**W-7750 Konstanz(DE)**
Erfinder: **Hanauer, Guido, Dr.**
**Hasenhof 7**
**W-7752 Reichenau(DE)**
Erfinder: **Flockerzi, Dieter, Dr.**
**Ackerweg 26**
**W-7753 Allensbach(DE)**

(54) **Neue Pyridazine.**

(57) Verbindungen der Formel I

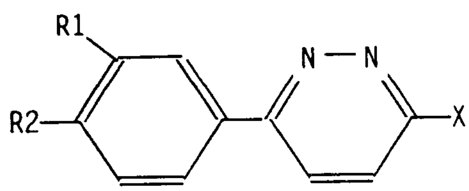

(I)

worin R1, R2 und X die in der Beschreibung angegebene Bedeutung haben, und ihre Salze und N-Oxide sind neue Wirkstoffe für die Behandlung von Bronchialerkrankungen und Dermatosen.

EP 0 510 562 A1

## Technisches Gebiet

Die Erfindung betrifft 3-Amino-6-aryl-pyridazine, Verfahren zu ihrer Herstellung, ihre Anwendung und sie enthaltende Arzneimittel. Die erfindungsgemäßen Verbindungen werden in der pharmazeutischen Industrie zur Herstellung von Medikamenten verwendet.

## Stand der Technik

Die beschriebenen 3-Amino-6-aryl-pyridazine sind neu.

## Beschreibung der Erfindung

Es wurde gefunden, daß die unten näher beschriebenen neuen Verbindungen vorteilhafte pharmakologische Eigenschaften aufweisen, durch die sie sich von bekannten Verbindungen in überraschender und besonders vorteilhafter Weise unterscheiden.

Gegenstand der Erfindung sind 3-Amino-6-aryl-pyridazine der allgemeinen Formel I (siehe das beigefügte Formelblatt), worin einer der Substituenten R1 und R2 Methoxy, Difluormethoxy oder Ethoxy, und der andere 1-5C-Alkoxy, 4-7C-Cycloalkoxy, 3-7C-Cycloalkylmethoxy, 3-5C-Alkenyloxy oder 1-4C-Polyfluoralkoxy bedeutet, X Amino bedeutet, und ihre Salze mit Säuren und ihre N-Oxide.

1-5C-Alkoxy ist geradkettig oder verzweigt. Als beispielhafte 1-5C-Alkoxyreste seien genannt der Methoxy-, Ethoxy-, n-Propoxy-, Isopropoxy-, n-Butoxy-, Isobutoxy-, sec.-Butoxy, tert.-Butoxy-, n-Pentyloxy, Isopentyloxy- und der 2,2-Dimethylpropoxyrest. 3-4C-Alkoxy ist bevorzugt.

4-7C-Cycloalkoxy steht beispielsweise für Cyclobutyloxy, Cyclopentyloxy, Cyclohexyloxy und Cycloheptyloxy, wovon Cyclopentyloxy bevorzugt ist.

3-7C-Cycloalkylmethoxy steht beispielsweise für Cyclopropylmethoxy, Cyclobutylmethoxy, Cyclopentylmethoxy, Cyclohexylmethoxy und Cycloheptylmethoxy, wovon Cyclopropylmethoxy und Cyclobutylmethoxy bevorzugt sind.

3-5C-Alkenyloxy ist geradkettig oder verzweigt. Die Doppelbindung von Alkenyloxy geht nicht von dem Kohlenstoffatom aus, das an das Sauerstoffatom bindet. Als beispielhafte 3-5C-Alkenyloxyreste seien genannt der Buten-2-yloxy, der Allyloxy- und der Methallyloxyrest.

1-5C-Alkoxy ist gegenüber 3-5C-Alkenyloxy bevorzugt.

Unter 1-4C-Polyfluoralkoxy wird geradkettiges oder verzweigtes 1-4C-Alkoxy verstanden, bei dem mindestens 2 Wasserstoffatome durch Fluor ersetzt sind. Geradkettiges 1-3C-Alkoxy, bei dem mindestens 2 Wasserstoffatome durch Fluor ersetzt sind, ist bevorzugt. Bevorzugte 1-4C-Polyfluoralkoxygruppen sind Trifluormethoxy, 1,1,2,2-Tetrafluorethoxy und insbesondere Difluormethoxy und 2,2,2-Trifluorethoxy.

Unter Amino wird die Gruppe $NH_2$ verstanden.

Als Salze kommen für Verbindungen der Formel I bevorzugt alle Säureadditionssalze in Betracht. Besonders erwähnt seien die pharmakologisch verträglichen Salze der in der Galenik üblicherweise verwendeten anorganischen und organischen Säuren. Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt. Als solche eignen sich beispielsweise wasserlösliche und wasserunlösliche Säureadditionssalze, wie das Hydrochlorid, Hydrobromid, Hydroiodid, Phosphat, Nitrat, Sulfat, Acetat, Citrat, Gluconat, Benzoat, Hibenzat, Fendizoat, Butyrat, Sulfosalicylat, Maleat, Laurat, Malat, Fumarat, Succinat, Oxalat, Tartrat, Amsonat, Embonat, Metembonat, Stearat, Tosilat, 3-Hydroxy-2-naphthoat oder Mesilat.

Die N-Oxide der Verbindungen der Formel I lassen sich durch die allgemeine Formel I* (siehe Formelblatt) beschreiben, in der R1, R2 und X die oben angegebenen Bedeutungen haben.

Die N-Oxide der erfindungsgemäßen 3-Amino-6-aryl-pyridazine können als tautomere Formen vorliegen. Ein Proton der 3-Aminogruppe vermag zwischen dieser Gruppe und dem Sauerstoff in 2-Stellung des Pyridazinrings zu wandern. Erfindungsgemäß ist bei Angabe oder Darstellung nur eines Tautomeren jeweils auch das andere Tautomere zu verstehen.

Eine Ausgestaltung (Ausgestaltung a) der Erfindung sind 3-Amino-6-aryl-pyridazine der allgemeinen Formel I, worin

R1 Methoxy, Difluormethoxy oder Ethoxy,
R2 1-4C-Alkoxy, 4-7C-Cycloalkoxy, 3-6C-Cycloalkylmethoxy, 3-4C-Alkenyloxy oder 1-2C-Polyfluoralkoxy und
X Amino bedeutet,

und ihre Salze mit Säuren und ihre N-Oxide.

Eine weitere Ausgestaltung (Ausgestaltung b) der Erfindung sind 3-Amino-6-aryl-pyridazine der allgemeinen Formel I, worin

R1 3-4C-Alkoxy, 4-7C-Cycloalkoxy, 3-6C-Cycloalkylmethoxy, 3-4C-Alkenyloxy oder 1-2C-Polyfluoralkoxy,
R2 Methoxy, Difluormethoxy oder Ethoxy und
X Amino bedeutet,

und ihre Salze mit Säuren und ihre N-Oxide.

Bevorzugte erfindungsgemäße Verbindungen sind solche der Formel I, worin einer der Substituenten R1 und R2 Methoxy, Difluormethoxy oder Ethoxy, und der andere 1-4C-Alkoxy, 4-7C-Cycloalkoxy, 3-6C-Cycloalkylmethoxy oder 1-2C-Polyfluoralkoxy bedeutet und X Amino bedeutet, und ihre Salze mit Säuren und ihre N-Oxide.

Bevorzugte Vertreter der Ausgestaltung a sind solche, in denen R2 1-4C-Alkoxy oder 1-2C-Polyfluoralkoxy bedeutet.

Bevorzugte Vertreter der Ausgestaltung b sind solche, in denen R1 3-4C-Alkoxy, 4-7C-Cycloalkoxy, 3-6C-Cycloalkylmethoxy oder 1-2C-Polyfluoralkoxy bedeutet.

Die Ausgestaltung b ist gegenüber der Ausgestaltung a bevorzugt.

Besonders bevorzugte erfindungsgemäße Verbindungen sind solche der Formel I, worin R1 2-4C-Alkoxy, Cyclopentyloxy, Cyclopropylmethoxy, Cyclobutylmethoxy, Difluormethoxy oder 2,2,2-Trifluorethoxy bedeutet, R2 Methoxy, Ethoxy oder Difluormethoxy bedeutet und X Amino bedeutet, und ihre pharmakologisch verträglichen Salze mit Säuren und ihre N-Oxide.

Gegenstand der Erfindung ist ferner ein Verfahren zur Herstellung der 3-Amino-6-aryl-pyridazine der allgemeinen Formel I, worin R1, R2 und X die oben angegebene Bedeutung haben, und ihrer Salze mit Säuren und ihrer N-Oxide, das dadurch gekennzeichnet ist, daß man

a) Verbindungen der Formel II (siehe beigefügtes Formelblatt ), worin R1 und R2 die oben angegebene Bedeutung besitzen und Y eine nucleophil verdrängbare Abgangsgruppe bedeutet, mit Ammoniak umsetzt, oder daß man

b) 3-Hydrazino-pyridazin der Formel III (siehe beigefügtes Formelblatt), worin R1 und R2 die oben angegebene Bedeutung haben, katalytisch hydriert, oder daß man

c) Phosphazene der Formel IV (siehe beigefügtes Formelblatt), worin R1 und R2 die oben angegebene Bedeutung haben, hydrolysiert, oder daß man

d) Benzylverbindungen der Formel V (siehe beigefügtes Formelblatt), worin R1 und R2 die oben angegebene Bedeutung haben, katalytisch hydriert,

und daß man gewünschtenfalls anschließend die nach a), b), c) oder d) erhaltenen Verbindungen I in ihre Salze überführt, oder daß man gewünschtenfalls anschließend aus erhaltenen Salzen der Verbindungen I die Verbindungen I freisetzt oder daß man gewünschtenfalls anschließend die nach a), b), c) oder d) erhaltenen Verbindungen I in ihre N-Oxide überführt.

Das Verfahren gemäß Variante a) wird auf eine dem Fachmann an sich vertraute Weise durchgeführt. Als nucleophil verdrängbare Abgangsgruppen Y kommen insbesondere Halogenatome, vor allem Chlor und Brom in Betracht. Die Umsetzung mit Ammoniak erfolgt beispielsweise entsprechend E.A. Steck et al., J.Heterocycl.Chem. **12**, 1009, 1975 in einem Alkohol, wie Ethanol, Ethylenglykol oder Polyethylenglykol 400, wobei vorzugsweise mit überschüssigem Ammoniak bei Temperaturen von 150 bis 200 °C, bevorzugt bei 180 bis 200 °C, in einem Autoklaven gearbeitet wird.

Beim Verfahren gemäß Variante b) werden die 3-Hydrazino-pyridazine der Formel III auf eine dem Fachmann an sich bekannte Weise hydriert. Beispielsweise werden die Verbindungen III in einem Alkohol, bevorzugt Methanol, mit einem Zusatz eines Hydrierungskatalysators, wie Raney-Nickel oder Palladium/Kohle bei Temperaturen zwischen 20 und 65 °C, bevorzugt bei Raumtemperatur hydriert. Alternativ können die Verbindungen III der katalytischen Transfer-Hydrogenolyse unterworfen werden, wie sie z.B. bei G. Brieger und T.J.Nestrick in Chem.Rev. 74, 567 (1974) beschrieben wird. Demgemäß werden die Verbindungen III zunächst mit einer Mineralsäure, wie Salz- oder Schwefelsäure, in ihre Salze überführt und anschließend in einem Alkohol, wie z.B. Methanol oder Ethanol, mit Palladium/Kohle und einem Wasserstoff-Donor, wie Cyclohexen, Cyclohexadien, Ameisensäure oder Ammoniumformiat bei Temperaturen von 20 bis 80 °C hydriert.

Die Hydrolyse der Phosphazene IV gemäß Verfahrensvariante c) erfolgt in an sich bekannter Weise, wobei bevorzugt in verdünnten Mineralsäuren, wie Salzsäure oder Schwefelsäure, oder in wäßrigen organischen Säuren, wie Essigsäure oder Ameisensäure gearbeitet wird.

Das Verfahren nach Verfahrensvariante d) wird ebenfalls in an sich bekannter Weise durchgeführt, wobei die Debenzylierung der Benzylverbindungen V bevorzugt unter den Bedingungen der katalytischen Transfer-Hydrogenolyse erfolgt, wozu die Verbindungen V beispielsweise in einer Mischung aus

Eisessig/konz. Salzsäure und Cyclohexen in Gegenwart von Palladium/Kohle als Katalysator debenzyliert werden.

Säureadditionssalze erhält man durch Auflösen der freien Base in einem geeigneten Lösungsmittel, z.B. in einem chlorierten Kohlenwasserstoff, wie Methylenchlorid oder Chloroform, oder einem niedermolekularen aliphatischen Alkohol (Ethanol, Isopropanol), das die gewünschte Säure enthält, oder dem die gewünschte Säure anschließend zugegeben wird.

Die Salze werden durch Filtrieren, Umfällen, Ausfällen mit einem Nichtlösungsmittel für das Anlagerungssalz oder durch Verdampfen des Lösungsmittels gewonnen. Erhaltene Salze können durch Alkalisierung, z.B. mit wäßriger Ammoniaklösung, in die freien Basen umgewandelt werden, welche wiederum in Säureadditionssalze übergeführt werden können. Auf diese Weise lassen sich pharmakologisch nicht verträgliche Säureadditionssalze in pharmakologisch verträgliche Säureadditionssalze umwandeln.

Die N-Oxidation wird auf eine dem Fachmann an sich vertraute Weise durchgeführt, wie sie beispielsweise von Eiji Ochiai in "Aromatic Amine Oxides", Seite 22 - 26, Elsevier Publishing Company, Amsterdam London New York, 1967, beschrieben ist. Als Oxidationsmittel kommen alle für die N-Oxidation üblicherweise verwendeten Reagenzien in Frage, insbesondere Wasserstoffperoxid oder (gegebenenfalls in situ hergestellte) organische und anorganische Peroxyverbindungen, wie z.B. Peroxyessigsäure, Trifluorperoxyessigsäure, 3,5-Dinitroperoxybenzoesäure, Peroxymaleinsäure, m-Chlorperoxybenzoesäure oder Kaliumpermanganat.

Die Verbindungen der Formel II sind bekannt oder sie können nach bekannten Verfahren hergestellt werden, wie sie z.B. in der EP-A-393500 beschrieben sind.

Die 3-Hydrazino-pyridazine der Formel III erhält man beispielsweise, indem man Verbindungen der Formel II entsprechend C.G. Wermuth et al., J.Med. Chem., **30,** 239, 1987, mit Hydrazinhydrat bei 100°C, bevorzugt in einem inerten Lösungsmittel, z.B. in einem Alkohol, wie 1-Butanol, oder ohne Lösungsmittel umsetzt.

Die Phosphazene der Formel IV erhält man ebenfalls ausgehend von Verbindungen der Formel II, die man zunächst entsprechend Th. Kappe et al., Synthesis 1989, 666, nach der Technik der Phasentransferkatalyse (siehe J.Dockx, Synthesis 1973, 441) zu Tetrazolo[1,5-b]pyridazinen umgesetzt.

Hierzu werden die Verbindungen II beispielsweise in einem Kohlenwasserstoff, wie z.B. Toluol oder Xylol, oder in einem Ether, wie z.B. Dioxan, oder einem Keton, wie z.B. 2-Methyl-4-pentanon (Isobutylmethylketon), oder einem N,N-disubstituierten Säureamid, wie z.B. Dimethylformamid oder N-Methylpyrrolidon unter wasserfreien Bedingungen, bevorzugt in Gegenwart von 0.1 bis 1.0 Mol des Phasentransferkatalysators bei Temperaturen von 50 bis 200°C, insbesondere von 80 bis 150°C, bevorzugt beim Siedepunkt des Lösungsmittels, mit mindestens 1 Mol Alkaliazid umgesetzt. Als Phasentransferkatalysatoren kommen die für nucleophile Verdrängungen, insbesondere für Halogenaustausch gebräuchlichen in Frage. Geeignet sind z.B. Kronenether, quartäre Phosphoniumsalze und insbesondere quartäre Ammoniumsalze, wie z.B. Tetrabutylammoniumchlorid.

Anschließend werden die erhaltenen Tetrazolo[1,5-b]pyridazine durch Erhitzen mit mindestens 1 Mol Triphenylphosphin in einem inerten Lösungsmittel, wie Benzol, Toluol, Xylol oder Chlorbenzol, bei 80 bis 150°C, insbesondere bei 80 bis 135°C, bevorzugt beim Siedepunkt des Lösungsmittels, in die Phosphazene IV übergeführt.

Die Verbindungen der Formel V werden aus den entsprechenden Verbindungen II durch Erhitzen mit Benzylamin, bevorzugt ohne Lösungsmittel bei Temperaturen von 140 bis 200°C, bevorzugt bei 180 bis 190°C hergestellt.

Für die Herstellung der Verbindungen der Ausgestaltungen a und b werden entsprechende Ausgangsverbindungen der allgemeinen Formel II, III, IV und V, worin R1 und R2 die oben jeweils angegebenen Bedeutungen haben, eingesetzt.

Die folgenden Beispiele dienen zur näheren Erläuterung der Erfindung. Fp. bedeutet Schmelzpunkt, Sdp. bedeutet Siedepunkt.

## Beispiele

**1.** 3-Amino-6-(3-methoxy-4-propoxyphenyl)pyridazin

10 g (35,9 mmol) 3-Chlor-6-(3-methoxy-4-propoxyphenyl)pyridazin werden in 50 ml Ethylenglykol, das bei 0°C mit Ammoniak gesättigt wurde, in einem Autoklaven 6 Stunden auf 220°C erhitzt. Nach dem Abkühlen wird das Reaktionsgemisch mit 500 ml Wasser verdünnt und der sich bildende Niederschlag abgesaugt. Nach dem Trocknen des Rohprodukts wird dieses durch Säulenchromatographie an Kieselgel mit Chloroform als Elutionsmittel gereinigt. Man erhält 7,6 g (81,7 %) der Titelverbindung vom Fp. 147°C.

**2.** 3-Amino-6-[4-methoxy-3-(2-methylpropoxy)phenyl]pyridazin

7,0 g (24 mmol) 3-Chlor-6-[4-methoxy-3-(2-methylpropoxy)phenyl]pyridazin werden wie im Beispiel 1 beschrieben in 40 ml mit Ammoniak gesättigtem Ethylenglykol umgesetzt. Das durch Fällung mit Wasser gewonnene Rohprodukt wird in Chloroform gelöst, die Lösung mit Kaliumcarbonat getrocknet und das Produkt mit Petrolether (Sdp.50-70 ° C) ausgefällt. Man erhält 3,5 g (53,8 %) der Titelverbindung vom Fp. 187 ° C.

Analog erhält man unter Einsatz entsprechender 3-Chlor-6-aryl-pyridazine:
3-Amino-6-[4-methoxy-3-(1-methylethoxy)phenyl]pyridazin Fp. 184 ° C (81,7 %)
3-Amino-6-[3-methoxy-4-(2-methylpropoxy)phenyl]pyridazin Fp. 140 ° C (76,1 %)
3-Amino-6-[3-methoxy-4-(1-methylethoxy)phenyl]pyridazin Fp. 153 ° C (89,8 %)
3-Amino-6-(3-cyclopentyloxy-4-methoxyphenyl)pyridazin Fp. 207 ° C (99,2 %)
3-Amino-6-(3-cyclohexyloxy-4-methoxyphenyl)pyridazin Fp. 199 ° C (72,3 %)
3-Amino-6-(3-cycloheptyloxy-4-methoxyphenyl)pyridazin Fp. 142 ° C (88,1 %)
3-Amino-6-[4-ethoxy-3-(2-methylpropoxy)phenyl]pyridazin Fp. 144 ° C (91,5 %)
3-Amino-6-(4-difluormethoxy-3-ethoxyphenyl)pyridazin Fp. 141 ° C (98,4 %)
3-Amino-6-(4-difluormethoxy-3-methoxyphenyl)pyridazin Fp. 187-188 ° C (86,5 %)
3-Amino-6-(3-difluormethoxy-4-ethoxyphenyl)pyridazin Fp. 170 ° C (96,7 %)
3-Amino-6-(3-cyclobutylmethoxy-4-methoxyphenyl)pyridazin Fp. 197 ° C (34,5 %)
3-Amino-6-(3-cyclopentyloxy-4-difluormethoxyphenyl)pyridazin Fp. 111-2 ° C (91,1 %)
3-Amino-6-[4-difluormethoxy-3-(1-methylethoxy)phenyl]pyridazin Fp. 153 ° C (100,0 %)
3-Amino-6-[4-difluormethoxy-3-(2,2,2-trifluorethoxy)phenyl]pyridazin Fp. 96 ° C (88,3 %)
3-Amino-6-[4-difluormethoxy-3-(2-methylpropoxy)phenyl]pyridazin Fp. 120-1 ° C (88,0 %)
3-Amino-6-(3-cyclopropylmethoxy-4-difluormethoxyphenyl)pyridazin Fp. 106 ° C (64,1 %)

**3.** 3-Amino-6-(3-cyclopentyloxy-4-methoxyphenyl)pyridazin

a) 14 g (46 mmol) 3-Chlor-6-(3-cyclopentyloxy-4-methoxyphenyl)pyridazinwerden in 74 g (0,69 mol) Benzylamin 3 Stunden auf 200 ° C erhitzt. Anschließend wird die Mischung auf Eis gegeben, mit Dichlormethan extrahiert und die organische Phase nach dem Trocknen über Kaliumcarbonat eingeengt. Man versetzt den Rückstand mit Petrolether (Sdp. 50-70 ° C), saugt den entstandenen Niederschlag ab und kristallisiert aus Essigester/Cyclohexan um. Man erhält 15,9 g (92,4 %) 3-Benzylamino-6-(3-cyclopentyloxy-4-methoxyphenyl)pyridazin vom Fp. 116 ° C.

b) 15 g (40 mmol) 3-Benzylamino-6-(3-cyclopentyloxy-4-methoxyphenyl)pyridazin werden in einer Mischung von 100 ml Eisessig, 6,5 ml konz. Salzsäure, 9,8 g (0.12 mol) Cyclohexen und 3 Spatelspitzen Pd/C (10-proz.) 4 Stunden zum Rückfluß erhitzt. Man fügt nochmals 9,8 g Cyclohexen und Pd/C hinzu und kocht weitere 16 Stunden. Der Katalysator wird abfiltriert, die Lösung mit 1 l Wasser verdünnt, mit konz. Ammoniak neutralisiert und der entstandene Niederschlag abgesaugt. Zur Reinigung wird das Produkt an Kieselgel mit Dichlormethan chromatographiert. Man erhält 4,8 g (42,1 %) 3-Amino-6-(3-cyclopentyloxy-4-methoxyphenyl)pyridazin vom Fp. 207 ° C.

**4.** 3-Amino-6-(4-difluormethoxy-3-methoxyphenyl)pyridazin

a) 5 g (17 mmol) 3-Chlor-6-(4-difluormethoxy-3-methoxyphenyl)pyridazin werden in 30 ml Benzylamin 1 Stunde auf 180 ° C erhitzt. Das Gemisch wird in 500 ml gesättigte Kaliumcarbonat-Lösung eingerührt, der entstandene Niederschlag abgesaugt, mit Wasser ausgewaschen, getrocknet und aus Essigester umkristallisiert. Man erhält 4,8 g (77,4 %) 3-Benzylamino-6-(4-difluormethoxy-3-methoxyphenyl)pyridazin vom Fp. 136 ° C.

b) 1,8 g (50,4 mmol) 3-Benzylamino-6-(4-difluormethoxy-3-methoxyphenyl)pyridazin werden in 30 ml Ethanol gelöst, mit 0,42 ml konz. Salzsäure versetzt und am Rotationsverdampfer bis zur Trockne eingedampft. Man löst das gebildete Hydrochlorid wieder in 30 ml Ethanol, fügt 0,95 g Ammoniumformiat und 0,5 g Palladium/Kohle (10-proz.) zu und kocht am Rückfluß. Der Fortgang der Debenzylierung wird dünnschichtchromatografisch verfolgt und gegebenenfalls weiteres Ammoniumformiat und Katalysator zugesetzt. Nach beendeter Reaktion wird der Katalysator abfiltriert, die Lösung mit 2 N Natronlauge alkalisch gestellt und mit Essigester extrahiert. Die organische Phase wird über geglühtem Kaliumcarbonat getrocknet, vom Trockenmittel abgesaugt, eingedampft und der Rückstand an Kieselgel mit Essigester chromatografiert. Nach dem Eindampfen der entsprechenden Fraktionen erhält man 0,9 g (69,2 %) 3-Amino-6-(4-difluormethoxy-3-methoxyphenyl)pyridazin vom Fp. 184 ° C.

**5.** 3-Amino-6-[4-methoxy-3-(1-methylethoxy)phenyl]pyridazin

a) 25 g (90 mmol) 3-Chlor-6-[4-methoxy-3-(1-methylethoxy)phenyl]pyridazin werden mit 22,5 g (0,45 mol) Hydrazinhydrat in 200 ml Butanol 12 Stunden am Rückfluß gekocht. Anschließend wird die Reaktionsmischung mit Eiswasser auf das drei- bis vierfache Volumen verdünnt, der Niederschlag abfiltriert und aus Isopropanol/Cyclohexan umkristallisiert. Man erhält 18,9 g (76,8 %) 3-Hydrazino-6-[4-methoxy-3-(1-methylethoxy)phenyl]pyridazin vom Fp. 116° C.

b) 10 g (36 mmol) 3-Hydrazino-6-[4-methoxy-3-(1-methylethoxy)phenyl]pyridazin werden in 100 ml Methanol in Anwesenheit von 1 g Raney-Nickel mit Wasserstoff unter gutem Rühren bei Normaldruck und Raumtemperatur hydriert. Nach 4 Stunden ist kein Ausgangsmaterial mehr nachzuweisen. Die Lösung wird vom Katalysator abfiltriert, im Vakuum eingeengt und der Rückstand aus Isopropanol/Cyclohexan kristallisiert. Man erhält 4,3 g (46 %) 3-Amino-6-[4-methoxy-3-(1-methylethoxy)-phenyl]pyridazin vom Fp. 184° C.

**6.** 3-Amino-6-(4-difluormethoxy-3-methoxyphenyl)pyridazin

a) 20,0 g (69,8 mmol) 3-Chlor-6-(4-difluormethoxy-3-methoxyphenyl)pyridazin werden in 100 ml n-Butanol mit 17 ml (350 mmol) Hydrazinhydrat 8 Stunden am Rückfluß gekocht. Die Lösung wird auf etwa 80° C abgekühlt und mit 100 ml gesättigter Soda-Lösung und 200 ml Wasser verrührt. Der Niederschlag wird abgesaugt, mit Wasser gewaschen und in Vakuum getrocknet. Man erhält 18,4 g (93,4 %) 3-Hydrazino-6-(4-difluormethoxy-3-methoxyphenyl)pyridazin vom Fp. 154° C.

b) 5,0 g (18 mmol) 3-Hydrazino-6-(4-difluormethoxy-3-methoxyphenyl)pyridazin werden in 50 ml Ethanol und 2,3 ml (27 mmol) konz. Salzsäure gelöst, mit 1 g Palladium/Kohle und 3,4 g (54 mmol) Ammoniumformiat versetzt und auf 80° C erhitzt. Nach beendeter Gasentwicklung wird das Gemisch noch 3 Stunden gerührt, vom Katalysator abfiltriert, die Lösung in 1 l Wasser eingerührt, mit konz. Ammoniak alkalisch gestellt und der entstandene Niederschlag auf einer Nutsche gesammelt. Nach Auswaschen mit Wasser wird der Filterkuchen getrocknet und in einer Mischung von Dichlormethan/Methanol 95:5 an Kieselgel chromatographiert. Die entsprechenden Fraktionen werden im Vakuum eingeengt. Man erhält 3 g (63,6 %) 3-Amino-6-(4-difluormethoxy-3-methoxyphenyl)pyridazin vom Fp. 184° C.

**7.** 3-Amino-6-(4-difluormethoxy-3-ethoxyphenyl)pyridazin-2-oxid

8,9 g (31,6 mmol) 3-Amino-6-(4-difluormethoxy-3-ethoxyphenyl)pyridazin werden in 60 ml Eisessig mit 10,9 g (63,2 mmol) m-Chlorperoxybenzoesäure unter Rühren 2 Std. auf 60° C erhitzt. Nach dem Abkühlen wird die Lösung in 100 ml Wasser eingerührt, die Lösung mit Essigester erschöpfend extrahiert, der organische Extrakt über Magnesiumsulfat getrocknet, in Vakuum eingeengt und an Kieselgel neutral zuerst mit Essigester, dann mit Essigester/Methanol 8:2 chromatografiert. Die entsprechenden Fraktionen werden eingeengt, der Rückstand in wenig Essigester aufgenommen, mit Petrolether (Sdp. 40-70° C) bis zur beginnenden Trübung versetzt und das Produkt unter Kühlung kristallisiert. Die Kristalle werden abgesaugt, mit Petrolether gewaschen und in Vakuum bei 80° C getrocknet. Man erhält 5,7 g (60,7 %) der Titelverbindung vom Fp. 202-3° C.

**8.** 3-Amino-6-(4-difluormethoxy-3-methoxyphenyl)pyridazin-2-oxid

20,0 g (74,8 mmol) 3-Amino-6-(4-difluormethoxy-3-methoxyphenyl)pyridazin werden in 100 ml Eisessig gelöst, unter Rühren mit 30,6 ml (300 mmol) 30 %igem Wasserstoffperoxid versetzt und die Mischung bei 80° C 2,5 Std. gerührt. Die Reaktionslösung wird mit 1 kg Eis und 200 ml konz. Ammoniaklösung verdünnt, der gebildete Niederschlag auf einer Nutsche gesammelt, zuerst mit Wasser, dann mit Ethanol und Petrolether (Sdp. 40-70° C) gewaschen und in Vakuum bei 75° C getrocknet. Man erhält 18,1 g (85,4 %) der Titelverbindung vom Fp. 214° C.

Analog erhält man unter Einsatz entsprechender 3-Amino-6-phenyl-pyridazine:
3-Amino-6-(3-difluormethoxy-4-ethoxyphenyl)pyridazin-2-oxid Fp. 188° C (53,4 %)
3-Amino-6-[4-difluormethoxy-3-(1-methylethoxy)phenyl]pyridazin-2-oxid Fp. 207° C (29,2 %)
3-Amino-6-[4-difluormethoxy-3-(2-methylpropoxy)phenyl]pyridazin-2-oxid Fp. 134-35° C (51,0 %)
3-Amino-6-(3-cyclopentyloxy-4-difluormethoxyphenyl)pyridazin-2-oxid Fp. 167° C (52,4 %)
3-Amino-6-[4-difluormethoxy-3-(2,2,2-trifluorethoxy)phenyl]pyridazin-2-oxid Fp. 222° C (42,3 %)
3-Amino-6-(3-cyclopropylmethoxy-4-difluormethoxyphenyl)pyridazin-2-oxid Fp. 159° C (86,4 %)

## Gewerbliche Anwendbarkeit

Die erfindungsgemäßen 3-Amino-6-aryl-pyridazine sowie ihre Salze und N-Oxide besitzen wertvolle pharmakologische Eigenschaften, die sie gewerblich verwertbar machen. Sie zeichnen sich vor allem durch solche Eigenschaften aus, die sie für die Therapie von Atemwegserkrankungen verschiedener Genese geeignet erscheinen lassen. Insbesondere können entzündliche und allergeninduzierte Bronchialerkrankungen aufgrund der antiinflammatorischen und broncholytischen Wirksamkeit der erfindungsgemäßen Verbindungen behandelt werden. Daneben zeichnen sich die erfindungsgemäßen Verbindungen durch eine geringe Toxizität, eine große therapeutische Breite und das Fehlen wesentlicher Nebenwirkungen aus.

Die broncholytische und antiinflammatorische Wirksamkeit der erfindungsgemäßen Verbindungen ermöglicht ihren Einsatz in der Human- und Veterinärmedizin, wobei sie zur Behandlung und Prophylaxe von Krankheiten, die auf Erkrankungen der Bronchien beruhen, verwendet werden. Beispielsweise können akute und chronisch obstruktive Atemwegserkrankungen verschiedener Genese (Bronchitis, allergische Bronchitis, Asthma bronchiale) bei Mensch und Tier behandelt werden.

Ein weiterer Gegenstand der Erfindung ist daher ein Verfahren zur Behandlung von Säugetieren einschließlich Menschen, die an einer der oben genannten Krankheiten erkrankt sind. Das Verfahren ist dadurch gekennzeichnet, daß man dem erkrankten Säugetier eine therapeutisch wirksame und pharmakologisch verträgliche Menge einer oder mehrer der erfindungsgemäßen Verbindungen verabreicht.

Weiterer Gegenstand der Erfindung sind die erfindungsgemäßen Verbindungen zur Anwendung bei der Behandlung und/oder Prophylaxe von Krankheiten, die auf Erkrankungen der Bronchien beruhen.

Ebenso betrifft die Erfindung die Verwendung der erfindungsgemäßen Verbindungen zur Herstellung von Arzneimitteln, die zur Behandlung und/oder Prophylaxe von Krankheiten, die auf Erkrankungen der Bronchien beruhen, eingesetzt werden.

Weiterhin sind Arzneimittel zur Behandlung und/oder Prophylaxe von Krankheiten, die auf Erkrankungen der Bronchien beruhen und die eine oder mehrere der erfindungsgemäßen Verbindungen und/oder ihre pharmakologisch verträglichen Salze enthalten, Gegenstand der Erfindung.

Die erfindungsgemäßen Arzneimittel werden nach an sich bekannten Verfahren hergestellt, wobei bezüglich der Zubereitungen, Dosierungen, Darreichungsformen etc. beispielsweise auf die Ausführungen im Europäischen Patent 163 965 verwiesen wird.

Die erfindungsgemäßen 3-Amino-6-aryl-pyridazine sowie ihre Salze und N-Oxide eignen sich weiterhin in hervorragender Weise zur Behandlung von Dermatosen.

Als Dermatosen seien insbesondere proliferative, entzündliche und allergische Hauterkrankungen erwähnt. So können die Verbindungen der Formel I beispielsweise zur Verhütung und Behandlung folgender Hauterkrankungen eingesetzt werden: Psoriasis vulgaris, toxisches und allergisches Kontaktekzem, atopisches Ekzem, seborrhoisches Ekzem, follikuläre und flächenhafte Pyodermien, endogene und exogene Akne, Akne rosacea sowie andere proliferative, entzündliche und allergische Hauterkrankungen. Weiterer Gegenstand der Erfindung ist somit die Verwendung von Verbindungen der Formel I und ihren pharmakologisch verträglichen Salzen und N-Oxiden zur Behandlung von solchen Individuen, die an Dermatosen erkrankt sind.

Die Anwendung der Verbindungen der Formel I erfolgt insbesondere in Form solcher Arzneimittel, die für eine topische Applikation geeignet sind. Für die Herstellung der Arzneimittel werden die Verbindungen der Formel I und/oder ihre pharmakologisch verträglichen Salze und/oder ihre N-Oxide ( = Wirkstoffe) vorzugsweise mit geeigneten pharmazeutischen Hilfsstoffen vermischt und zu geeigneten Arzneiformulierungen weiterverarbeitet. Als geeignete Arzneiformulierungen seien beispielsweise Puder, Emulsionen, Suspensionen, Sprays, Öle, Salben, Fettsalben, Cremes, Pasten, Gele oder Lösungen genannt, in denen der Wirkstoffgehalt vorteilhafterweise zwischen 0,1 und 99 % beträgt.

Welche Hilfsstoffe für die gewünschten Arzneiformulierungen geeignet sind, ist dem Fachmann aufgrund seines Fachwissens geläufig. Neben Lösemitteln, Gelbildnern, Salbengrundlagen und anderen Wirkstoffträgern können beispielsweise Antioxidantien, Dispergiermittel, Emulgatoren, Konservierungsmittel, Lösungsvermittler oder Permeationspromotoren verwendet werden.

Außerdem eignen sich die erfindungsgemäßen 3-Amino-6-aryl-pyridazine sowie ihre Salze und N-Oxide zur Verhütung und Behandlung von Krankheitszuständen, die durch bestimmte Zytokine (insbesondere durch Interleukine und vor allem durch den Tumornekrosefaktor) sowie Leukotriene ausgelöst werden, wobei die Behandlung des septischen Schocks bzw. des toxischen Schocksyndroms besonders hervorzuheben ist.

Weiterhin können die erfindungsgemäßen 3-Amino-6-aryl-pyridazine sowie ihre Salze und N-Oxide zur Verhütung und Behandlung von allergischen und/oder chronischen Fehlreaktionen im Bereich der oberen Atemwege (Rachenraum, Nase) und der angrenzenden Regionen (Nasennebenhöhlen, Auge), wie z.B. der

allergischen Rhinitis/Sinusitis, chronischen Rhinitis/Sinusitis, allergischen Conjunctivitis und von Nasenpolypen eingesetzt werden.

## Biologische Untersuchungen

In den anschließenden Tabellen sind die untersuchten Verbindungen durch Nummern gekennzeichnet, die wie folgt zugeordnet sind:

1 3-Amino-6-(3-methoxy-4-propoxyphenyl)pyridazin
2 3-Amino-6-[4-methoxy-3-(2-methylpropoxy)phenyl]pyridazin
3 3-Amino-6-[4-methoxy-3-(1-methylethoxy)phenyl]pyridazin
4 3-Amino-6-[3-methoxy-4-(2-methylpropoxy)phenyl]pyridazin
5 3-Amino-6-[3-methoxy-4-(1-methylethoxy)phenyl]pyridazin
6 3-Amino-6-(3-cyclopentyloxy-4-methoxyphenyl)pyridazin
7 3-Amino-6-(3-cyclohexyloxy-4-methoxyphenyl)pyridazin
8 3-Amino-6-(3-cycloheptyloxy-4-methoxyphenyl)pyridazin
9 3-Amino-6-[4-ethoxy-3-(2-methylpropoxy)phenyl]pyridazin
10 3-Amino-6-(4-difluormethoxy-3-ethoxyphenyl)pyridazin
11 3-Amino-6-(4-difluormethoxy-3-methoxyphenyl)pyridazin
12 3-Amino-6-(3-difluormethoxy-4-ethoxyphenyl)pyridazin
13 3-Amino-6-(3-cyclobutylmethoxy-4-methoxyphenyl)pyridazin
14 3-Amino-6-(3-cyclopentyloxy-4-difluormethoxyphenyl)pyridazin
15 3-Amino-6-[4-difluormethoxy-3-(1-methylethoxy)phenyl]pyridazin
16 3-Amino-6-[4-difluormethoxy-3-(2,2,2-trifluorethoxy)pyridazin
17 3-Amino-6-[4-difluormethoxy-3-(2-methylpropoxy)phenyl]pyridazin
18 3-Amino-6-(3-cyclopropylmethoxy-4-difluormethoxyphenyl)pyridazin
19 3-Amino-6-(4-difluormethoxy-3-ethoxyphenyl)pyridazin-2-oxid
20 3-Amino-6-(4-difluormethoxy-3-methoxyphenyl)pyridazin-2-oxid
21 3-Amino-6-(3-difluormethoxy-4-ethoxyphenyl)pyridazin-2-oxid
22 3-Amino-6-[4-difluormethoxy-3-(1-methylethoxy)phenyl]pyridazin-2-oxid
23 3-Amino-6-[4-difluormethoxy-3-(2-methylpropoxy)phenyl]pyridazin-2-oxid
24 3-Amino-6-(3-cyclopentyloxy-4-difluormethoxyphenyl)pyridazin-2-oxid
25 3-Amino-6-[4-difluormethoxy-3-(2,2,2-trifluorethoxy)phenyl]pyridazin-oxid
26 3-Amino-6-(3-cyclopropylmethoxy-4-difluormethoxyphenyl)pyridazin-2-oxid

Die bronchospasmolytische Wirkung der Verbindungen auf die Trachealspangen-Kette des Meerschweinchens wurde in vitro wie folgt geprüft:
Vier parallele, aus jeweils 6 Einzelringen bestehende Trachealspangen-Ketten des Meerschweinchens (weibl. und männl., 430 - 600 g) im Organbad [5 ml, Krebs-Henseleit-Lösung mit Zusatz von Phentolamin ($10^{-5}$ mol/l), 37 ° C, Vorspannung der Organe 2 g, Begasung mit Carbogen] entwickeln nach etwa 20 bis 30 Minuten eine stabile, tonische Spontankontraktur. An diesen dauerkontrahierten Organen kann unter isometrischen Meßbedingungen durch Applikation der Prüfsubstanz in kumulativ halblogarithmisch ansteigender Konzentration (z.B. $1x10^{-6}$ + $2x10^{-6}$ + $7x10^{-6}$ + $2x10^{-5}$ usw. mol/l) eine Relaxation herbeigeführt werden, wobei nach jeder Einzeldosis der Testsubstanz eine konstante Relaxations-Antwort abgewartet wird, bevor die nächst höhere Konzentration appliziert wird. Über einen Zeitraum von 20 bis 30 Minuten wird somit eine vollständige Dosis-Wirkungskurve der Testsubstanz erhalten. Die jeweilige Relaxation wird als Prozentbruchteil der durch Gabe von (-)Isoprenalin ($10^{-6}$ mol/l) maximal erreichbaren Relaxation ausgedrückt. Als Maß für die bronchodilatorische Aktivität dient die Konzentration der Testsubstanz, welche 50 % der maximal erreichbaren Relaxation bewirkt, ausgedrückt durch den negativen Logarithmus der $EC_{50}$ mol/l: -lg[$EC_{50}$].

In der Tabelle 1 sind die Werte -lg[$EC_{50}$] und die Quotienten aus den $EC_{50}$-Werten für Theophyllin und die untersuchte Substanz angegeben. Die gefundenen Werte zeigen eine große Überlegenheit der erfindungsgemäßen Verbindungen gegenüber Theophyllin bezüglich der bronchospasmolytischen Aktivität.

Tabelle 1

| Lfd.Nr. | -lg[$EC_{50}$] | [$EC_{50}$]$_{Theophyllin}$/ [$EC_{50}$]$_{Substanz}$ |
|---|---|---|
| 6 | 5,18 | 20,4 |
| 7 | 5,03 | 14,5 |
| 8 | 5,39 | 33,2 |
| 9 | 5,01 | 13,8 |
| 10 | 5,08 | 16,2 |
| 11 | 5,31 | 27,6 |
| 12 | 5,35 | 30,2 |
| 14 | 5,18 | 20,4 |
| 19 | 6,82 | 893 |
| 20 | 6,35 | 302 |
| 21 | 6,25 | 240 |
| 22 | 5,52 | 44,7 |
| 23 | 5,52 | 44,7 |
| 24 | 5,69 | 65,5 |
| 25 | 6,45 | 380 |
| 26 | 6,00 | 135 |
| Theophyllin | 3,87 | 1,0 |

Die bronchospasmolytische Wirkung wurde weiterhin am Modell "Histamininduzierter Bronchospasmus am narkotisierten Meerschweinchen" bestimmt:
Bei diesem Modell werden pharmakodynamische bzw. toxische Effekte an inneren sensiblen Rezeptoren, auf die Atmung und am Herz-Kreislauf-System vom Meerschweinchen simultan registriert [U. Kilian, E. Müller, E. Ch. Dittmann und J. Hamacher, Arzneimittel-Forschung 28 (II) 1699-1708, 1978]. An narkotisierten (Ethylurethan 1,25 g/kg i.p.) monovagotomierten, spontanatmenden Meerschweinchen (männl., 350 - 450 g) wurde das Pneumotachogramm registriert und zur Charakterisierung des durch Histamin (0,09 - 0,18 µmol/kg i.v.) ausgelösten Bronchospasmus die maximale Strömungsgeschwindigkeit der Atemluft während der Exspiration ($Vmax_e$) gemessen.

Ein Histaminspasmus vor Substanzgabe wurde mit mehreren Histaminspasmen nach Substanzgabe verglichen. Die Prüfsubstanzen wurden intravenös (i.v.) und/oder intrajejunal (i.j.) appliziert.

Es wurde gefunden, daß die untersuchten Verbindungen den histamin-induzierten Bronchospasmus am narkotisierten Meerschweinchen etwa 2 - 5 mal stärker hemmen als Theophyllin.

Tabelle 2

| Mittlere prozentuale bronchspasmolytische Wirkung 0-1 Std. p.appl. und prozentuale bronchospasmolytische Wirkung nach 1 Std. p.appl., gemessen an der Hemmung der histamininduzierten Abnahme von $Vmax_e$ | | | | | |
|---|---|---|---|---|---|
| Lfd.Nr. | Dosis µmol/kg | % Hemmung (0-1Std.) | | % Hemmung nach 1 Std | |
| | | i.v.- | i.j.-Gabe | i.v.- | i.j.-Gabe |
| 3 | 20 | 34 | | 20 | |
| 6 | 20 | 22 | | 15 | |
| 9 | 20 | 16 | | 20 | |
| 10 | 20 | 38 | | 44 | |
| 11 | 20 | 30 | | 26 | |
| 18 | 20 | 31 | | 35 | |
| | 10 | 33 | 32 | 38 | 43 |
| | 5 | 40 | | 49 | |
| 19 | 20 | 71 | | 81 | |
| 20 | 20 | 40 | | 48 | |
| 25 | 20 | 34 | | 33 | |
| 26 | 20 | 15 | | 12 | |
| Theophyllin | 100 | 34 | 45 | 22 | 44 |
| | 60 | 25 | 37 | 12 | 42 |
| | 20 | 13 | 11 | 5 | 8 |

Zusätzlich wurde die bronchospasmolytische Wirkung am Modell "Schutzwirkung gegen den Acetylcholin-induzierten Bronchospasmus am wachen Meerschweinchen" geprüft:
Die Versuchsführung erfolgt in Anlehnung an T. Olsson, Acta Allergologica 26, 438-447 (1971). Meerschweinchen (250 - 350 g) werden in einem verschlossenen Plexiglaszylinder (Volumen 5 l) vor Substanzgabe zweimal im Abstand von 20 Minuten nach Substanzgabe einem Acetylcholin-Nebel (0,06 % in 0,9 % Natriumchloridlösung; Ultraschallvernebler Heyer Use 77) ausgesetzt. Die Zeit vom Beginn der Verneblung bis zum Einsetzen deutlicher Atemanstrengungen (unter Umständen hypoxischer Krampfanfall in Seitenlage) wird gemessen und als Latenzzeit bezeichnet. Eine Verlängerung der Latenzzeit nach Substanzgabe auf mindestens die dreifache mittlere Latenzzeit vor Substanzgabe wird als Schutzwirkung angesehen und die Anzahl der in dem Kollektiv geschützten Tiere ermittelt. Die Applikation der Prüfsubstanzen erfolgt oral mittels Schlundsonde (Dosis 100 µmol/kg, Volumen 1 ml/kg, Suspensionsmittel 4 %ige Methocelsuspension in 0,9 %iger Natriumchloridlösung).

Im Kontrollversuch (ohne Substanzapplikation) liegt die Latenzzeit bei 2 Minuten. Die Applikation der Prüfsubstanz erfolgt per oral mittels Schlundsonde (Standarddosis 100 µmol, Volumen 1 ml 4 %ige Methocelsuspension in 0,9 %iger Natriumchloridlösung/kg). Nach 30 Minuten werden die Tiere erneut dem Acetylcholin-Nebel ausgesetzt und die Latenzzeiten gemessen. Eine Verlängerung der Latenzzeit auf mindestens die dreifache Länge wird als Schutzwirkung angesehen.

Tabelle 3

Schutzwirkung gegen den Acetylcholin-induzierten Bronchospasmus am wachen Meerschweinchen, ermittelt 30 Minuten nach oraler Substanz- bzw. Placebo-Gabe. Angegeben ist die prozentuale Anzahl geschützter Tiere im Vergleich zur Kontrollgruppe.

| Lfd.Nr. | Dosis µmol/kg | % Schutzwirkung |
|---|---|---|
| 2 | 30 | 50 |
| | 100 | 80 |
| 3 | 30 | 50 |
| | 100 | 70 |
| | 300 | 80 |
| 4 | 100 | 35 |
| 5 | 100 | 45 |
| 6 | 30 | 35 |
| | 100 | 85 |
| 7 | 100 | 35 |
| 8 | 100 | 50 |
| 9 | 100 | 65 |
| 10 | 100 | 50 |
| 11 | 100 | 80 |
| 12 | 100 | 50 |
| 13 | 100 | 65 |
| 18 | 10 | 75 |
| | 100 | 75 |
| 19 | 4 | 50 |
| 20 | 2 | 50 |
| 22 | 100 | 60 |
| 24 | 100 | 60 |
| 25 | 10 | 45 |
| | 100 | 70 |
| 26 | 10 | 70 |
| Theophyllin | 100 | 20 |
| | 200 | 40 |
| | 400 | 70 |

Als besonders aussagekräftig für eine zu erwartende bronchospasmolytische und/oder antiinflammatorische Wirkung wird die spezifische Hemmung der Phosphodiesterase IV (PDE IV = hoch affine cAMP-PDE durch cGMP nicht hemmbar, Rolipram-sensitiv) angesehen. Die Spezifizität der Hemmung wird dabei durch den Quotienten aus $[IC_{50}]_{PDE-III}/[IC_{50}]_{PDE-IV}$ (PDE III = hoch affine cAMP-PDE durch cGMP hemmbar) beschrieben [H.Hidaka et al., Adv.Cycl.Nucl. Res. 13, 145 (1984); TiPS 5, 237 (1984); R.Weishaar et al., J.Med.Chem. 28, (1985); S.A.Harrison et al., Molec. Pharmac. 29, 506 (1986); J.Klein-Tebbe et al., Allergologie 12, 12 (1989); C.Schudt et al., Allergologie 12, 12 (1989); C.Schudt et al., Agents and Action 1991 im Druck; C.D.Nicholson et al., TiPS 12, (1), 19 (1991)].

Deshalb wurde die PDE-Hemmung der erfindungsgemäßen Verbindungen an einer aus humanen Thrombozyten isolierten PDE III bzw. aus humanen neutrophilen polymorphkernigen Zellen (PMN's) sowie aus der Hundetrachea isolierten PDE IV bestimmt. Die Phosphodiesterasen III und IV werden nach Polson et al., Biochem. Pharmacol. 31, 3403-3406 (1982) chromatographisch isoliert.

Die Substanzen werden in DMSO gelöst und weiter verdünnt. Aus einer Reihe von bis hundertfach verdünnten Lösungen werden jeweils 2,1 µl vorgelegt und mit 212 µl Reaktionsgemisch versetzt. Das Reaktionsgemisch enthält Hepes (100 mmol/l), DTE (5 mmol/l), $MgCl_2$ (5 mmol/l), $CaCl_2$ (10 µmol/l), BSA Fraktion V 0,5 mg/ml, cAMP 0,5 µmol/l, 2,8 - $^3$H-cAMP 250000 cpm/ml (0,3 µCi/ml, s.A. 33,5 µCi/mmol), SV (snake venom) 25 µg/212 µl Testansatz).

In der Tabelle 4 sind die negativen Logarithmen der gefundenen $IC_{50}$Werte und die Quotienten aus den für die PDE III- und PDE IV-Hemmung ermittelten $IC_{50}$-Werten der erfindungsgemäßen Substanzen aufgeführt. Die erfindungsgemäßen Substanzen hemmen die PDE IV bedeutend spezifischer und stärker als Theophyllin.

Tabelle 4

| Hemmung der PDE III und PDE IV | | | | | |
|---|---|---|---|---|---|
| Lfd. Nr. | PDE III hum.Thromb. -lg[$IC_{50}$] | PDE IV Hund Trachea -lg[$IC_{50}$] | PDE IV hum.PMN's -lg[$IC_{50}$] | [$IC_{50}$]PDE III/ [$IC_{50}$]PDE IV Hund-Tr. | [$IC_{50}$]PDE III/ [$IC_{50}$]PDE IV PMN's |
| 2 | 3,68 | <5,0 | | < 20,9 | |
| 3 | 3,66 | 5,30 | | 43,7 | |
| 6 | <4,0 | 6,48 | 6,18 | > 304 | > 151 |
| 7 | 4,22 | 5,17 | | 8,9 | |
| 8 | 4,11 | 5,37 | | 18,2 | |
| 9 | 3,90 | 5,68 | | 60,2 | |
| 10 | 3,65 | 5,37 | | 52,5 | |
| 11 | 3,71 | 5,09 | | 22,9 | |
| 12 | 3,50 | 4,42 | | 9,6 | |
| 13 | 3,65 | 4,88 | | 17,0 | |
| 14 | 4,27 | | 7,15 | | 1466 |
| 15 | 3,97 | | 6,78 | | 794 |
| 16 | 3,48 | | 6,88 | | 2515 |
| 17 | 3,82 | | 6,96 | | 1466 |
| 18 | 3,84 | | 7,47 | | 4265 |
| 19 | 4,20 | 5,82 | 6,58 | 41,7 | 240 |
| 20 | 4,83 | 5,08 | 5,89 | 1,8 | 11,5 |
| 21 | 4,35 | - | 5,19 | - | 6,9 |
| 22 | 4,92 | - | 6,26 | - | 21,9 |
| 23 | 3,90 | - | 7,03 | - | 1350 |
| 24 | 4,32 | - | 7,24 | - | 832 |
| 25 | 4,15 | - | 6,42 | - | 186 |
| 26 | 4,74 | - | 7,48 | - | 550 |
| Theophyllin | 3,82 | | 3,77 | | 0,3 |

**Patentansprüche**

**1.** 3-Amino-6-aryl-pyridazine der allgemeinen Formel I

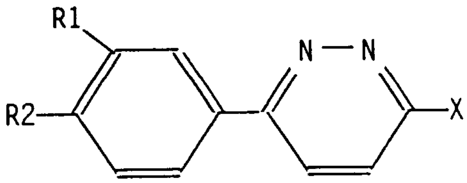

(I)

worin einer der Substituenten R1 und R2 Methoxy, Difluormethoxy oder Ethoxy, und der andere 1-5C-Alkoxy, 4-7C-Cycloalkoxy, 3-7C-Cycloalkylmethoxy, 3-5C-Alkenyloxy oder 1-4C-Polyfluoralkoxy bedeutet, X Amino bedeutet, und ihre Salze mit Säuren und ihre N-Oxide.

**2.** 3-Amino-6-aryl-pyridazine der allgemeinen Formel I nach Anspruch 1, worin
R1 Methoxy, Difluormethoxy oder Ethoxy,
R2 1-4C-Alkoxy, 4-7C-Cycloalkoxy, 3-6C-Cycloalkylmethoxy, 3-4C-Alkenyloxy oder 1-2C-Polyfluoralkoxy und
X Amino bedeutet,
und ihre Salze mit Säuren und ihre N-Oxide.

**3.** 3-Amino-6-aryl-pyridazine der allgemeinen Formel I nach Anspruch 1, worin
R1 3-4C-Alkoxy, 4-7C-Cycloalkoxy, 3-6C-Cycloalkylmethoxy, 3-4C-Alkenyloxy oder 1-2C-Polyfluoralkoxy,
R2 Methoxy, Difluormethoxy oder Ethoxy und
X Amino bedeutet,
und ihre Salze mit Säuren und ihre N-Oxide.

**4.** Verbindungen der Formel I nach Anspruch 1, worin einer der Substituenten R1 und R2 Methoxy, Difluormethoxy oder Ethoxy, und der andere 1-4C-Alkoxy, 4-7C-Cycloalkoxy, 3-6C-Cycloalkylmethoxy oder 1-2C-Polyfluoralkoxy bedeutet und X Amino bedeutet, und ihre Salze mit Säuren und ihre N-Oxide.

**5.** Verbindungen der Formel I nach Anspruch 1, worin R1 2-4C-Alkoxy, Cyclopentyloxy, Cyclopropylmethoxy, Cyclobutylmethoxy, Difluormethoxy oder 2,2,2-Trifluorethoxy bedeutet, R2 Methoxy, Ethoxy oder Difluormethoxy bedeutet und X Amino bedeutet, und ihre pharmakologisch verträglichen Salze mit Säuren und ihre N-Oxide.

**6.** Verfahren zur Herstellung der 3-Amino-6-aryl-pyridazine der allgemeinen Formel I nach Anspruch 1, worin R1, R2 und X die in Anspruch 1 angegebene Bedeutung haben, und ihrer Salze mit Säuren und ihrer N-Oxide, dadurch gekennzeichnet, daß man
a) Verbindungen der Formel II,

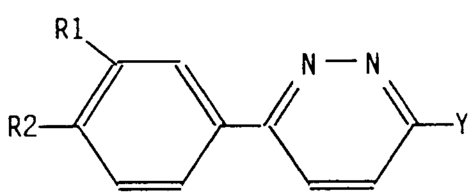

(II)

worin R1 und R2 die in Anspruch 1 angegebene Bedeutung besitzen und Y eine nucleophil verdrängbare Abgangsgruppe bedeutet, mit Ammoniak umsetzt, oder daß man
b) 3-Hydrazino-pyridazin der Formel III,

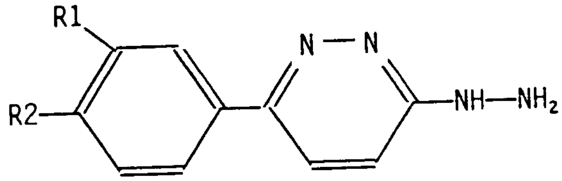

(III)

worin R1 und R2 die in Anspruch 1 angegebene Bedeutung haben, katalytisch hydriert, oder daß man
c) Phosphazene der Formel IV,

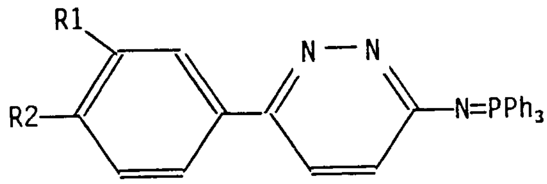

(IV)

worin R1 und R2 die in Anspruch 1 angegebene Bedeutung haben, hydrolysiert, oder daß man
d) Benzylverbindungen der Formel V,

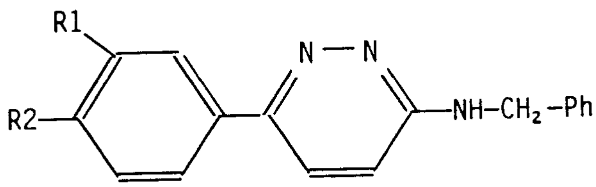

(V)

worin R1 und R2 die in Anspruch 1 angegebene Bedeutung haben, katalytisch hydriert,
und daß man gewünschtenfalls anschließend die nach a), b), c) oder d) erhaltenen Verbindungen I in ihre Salze überführt, oder daß man gewünschtenfalls anschließend aus erhaltenen Salzen der Verbindungen I die Verbindungen I freisetzt, oder daß man gewünschtenfalls anschließend die nach a), b), c) oder d) erhaltenen Verbindungen I in ihre N-Oxide überführt.

**7.** Arzneimittel enthaltend eine oder mehrere Verbindungen nach Anspruch 1.

**8.** Verbindungen nach Anspruch 1 zur Anwendung bei der Behandlung und/oder Prophylaxe von Erkrankungen der Bronchien.

**9.** Verwendung der Verbindungen nach Anspruch 1 zur Herstellung von Arzneimitteln zur Behandlung und/oder Prophylaxe von Erkrankungen der Bronchien.

**10.** Verbindungen nach Anspruch 1 zur Anwendung bei der Behandlung und/oder Prophylaxe von Dermatosen bei Säugern, insbesondere Menschen.

**11.** Verbindungen nach Anspruch 1 zur Anwendung bei der Behandlung und/oder Prophylaxe von proliferativen, entzündlichen oder allergischen Hauterkrankungen bei Säugern, insbesondere Menschen.

**12.** Verbindungen nach Anspruch 1 zur Anwendung bei der Behandlung und/oder Prophylaxe von Psoriasis oder atopischer Dermatitis bei Säugern, insbesondere Menschen.

**13.** Verwendung der Verbindungen nach Anspruch 1 zur Herstellung von Arzneimitteln für die Behandlung

von Dermatosen.

## Formelblatt

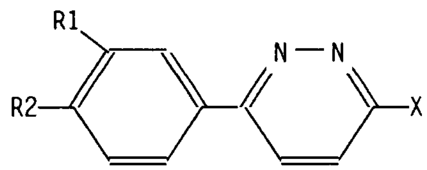

(I)

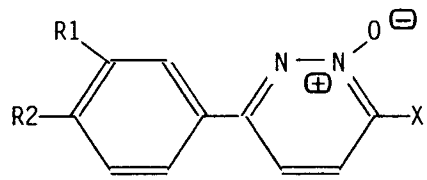

(I*)

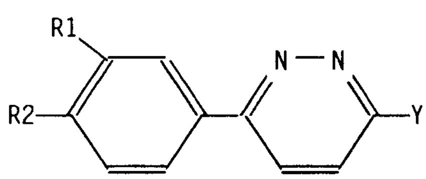

(II)

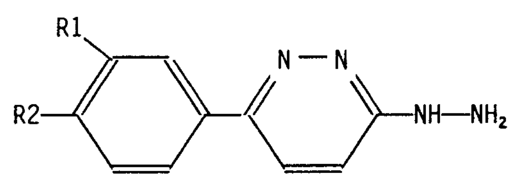

(III)

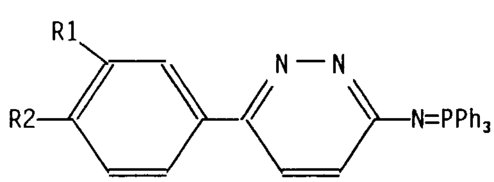

(IV)

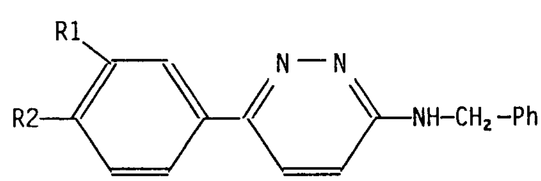

(V)

Europäisches Patentamt

# EUROPÄISCHER RECHERCHENBERICHT

Nummer der Anmeldung

EP 92 10 6743

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | EP-A-0 393 500 (BYK GULDEN LOMBERG CHEMISCHE FABRIK)<br>* das ganze Dokument *<br>----- | 1,2,7-13 | C07D237/20<br>A61K31/50 |
| | | | **RECHERCHIERTE SACHGEBIETE (Int. Cl.5 )** |
| | | | C07D |

Der vorliegende Recherchenbericht wurde für alle Patentansprüche erstellt

| Recherchenort | Abschlußdatum der Recherche | Prüfer |
|---|---|---|
| DEN HAAG | 30 JUNI 1992 | FRANCOIS J.C. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)